# EUROPÄISCHE PATENTSCHRIFT

(11) **EP 0 849 258 B1**
(45) Veröffentlichungstag und Bekanntmachung des Hinweises auf die Patenterteilung: **31.10.2001**
(21) Anmeldenummer: 97122313.6
(22) Anmeldetag: 17.12.1997
(51) Int. Cl.: C07C 263/04, C07C 265/06

(54) **Verfahren zur Herstellung von ethylenisch ungesättigten Isocyanaten**
Process for the preparation of ethylenically unsaturated isocyanates
Procédé de préparation d'isocyanates insaturés ethyléniques

(30) Priorität: 19.12.1996 EP 96810882
(43) Veröffentlichungstag der Anmeldung: 24.06.1998
(73) Patentinhaber: Novartis AG, 4056 Basel (CH)
(72) Erfinder: Lohmann, Dieter, 4142 Münchenstein (CH); Duthaler, Rudolf, 4126 Bettingen (CH)

(56) Entgegenhaltungen:
- DE-A- 4 011 914
- JP-A- 2 129 163
- US-A- 4 510 298
- US-A- 4 704 466
- DATABASE WPI Week 7013 Derwent Publications Ltd., London, GB; AN 70-20886 XP002029666 & SU 239 945 A (MIRONOV VF KOZYUKOV VP SH)

## Beschreibung

Die vorliegende Erfindung betrifft ein Verfahren zur Herstellung von ethylenisch ungesättigten Isocyanaten, insbesondere von Vinylisocyanaten, Vinylbenzylisocyanaten oder Isocyanatoestern von ungesättigten organischen Säuren wie z.B. von Isocyanatoalkyl(meth)acrylaten.

US-A-4 704 466 beschreibt ein Verfahren zur Herstellung von α-lsocyanato-substituierten Acrylsäureestern, worin ein α-Urethan-Acrylsäureester am Urethan-Stickstoff silyliert und das erhaltene N-Silyl-Urethan mittels Thermolyse bei Temperaturen von 150-300°C, vorzugsweise 250-300°C, zur α-lsocyanato-Verbindung umsetzt wird. Weiterhin ist aus Derwent Abstract Nr. AN 70-20886R ein Verfahren zur Herstellung von organischen Isocyanaten bekannt, worin wiederum ein Urethan am Urethan-Stickstoff silyliert und anschliessend mittels Thermolyse bei Temperaturen von 150 bis 210°C, vorzugsweise 190°C, zum Isocyanat umgesetzt wird.
lsocyanatoalkyl-(meth)acrylate sind bekannte Verbindungen und werden, mit z.B. in JP-A-02/129 163 offenbart, üblicherweise unter Einsatz von Phosgen hergestellt. So verläuft eine technische Synthese von Isocyanatoethyl-methacrylat (IEM) dergestalt, dass man aus der Reaktion von Ethanolamin mit Methylmethacrylat gewonnenes N-2-Hydroxyethyl-methacrylamid in Sulfolan bei Temperaturen von über 200°C zu 2-Isopropenyl-oxazolin cyclisiert und die erhaltene Verbindung mit Phosgen zur Isocyanatoverbindung umsetzt. Verschiedene Ansätze, das toxische und nur mit hohem apparativem Aufwand zu handhabende Phosgen zu umgehen, haben bisher nicht den gewünschten Erfolg gebracht. Es besteht daher Bedarf nach einer Phosgen-freien, schonenden Synthese von Isocyanatoalkyl-(meth)acrylaten und verwandten Verbindungen. Es wurde nun überraschend gefunden, dass man bestimmte ethylenisch ungesättigte Isocyanate vorteilhaft durch N-Silylierung entsprechender Urethan-Vorläufer und anschliessende thermische Behandlung bei milden Bedingungen erhalten kann.

Gegenstand der vorliegenden Erfindung ist daher ein Verfahren zur Herstellung von ethylenisch ungesättigten Isocyanaten der Formel 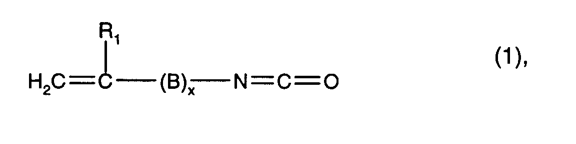
worin R₁ für Wasserstoff oder Methyl steht,
x die Zahl 0 oder 1 bedeutet,
B gegebenenfalls durch C₁-C₄-Alkyl oder C₁-C₄-Alkoxy substituiertes Phenylen oder C₇-C₁₂-Aralkylen oder ein Rest der Formel 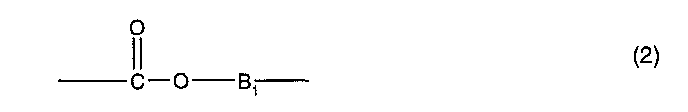 ist und B₁ lineares oder verzweigtes C₂-C₁₂-Alkylen, welches gegebenenfalls durch ein oder mehrere Sauerstoffatome unterbrochen ist, bedeutet, dadurch gekennzeichnet, dass man ein ethylenisch ungesättigtes Urethan der Formel 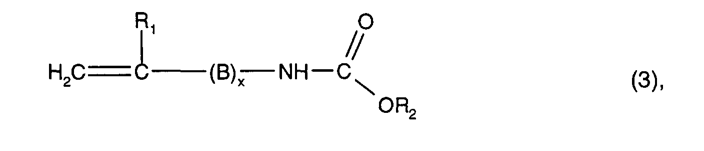 worin R₂ C₁-C₄-Alkyl oder gegebenenfalls durch C₁-C₄-Alkyl, C₁-C₄-Alkoxy oder Halogen substituiertes Phenyl ist und die Variablen R₁, B und x die oben angegebene Bedeutung haben, mit einem Silylierungsmittel in ein ethylenisch ungesättigtes N-Silyl-Urethan der Formel 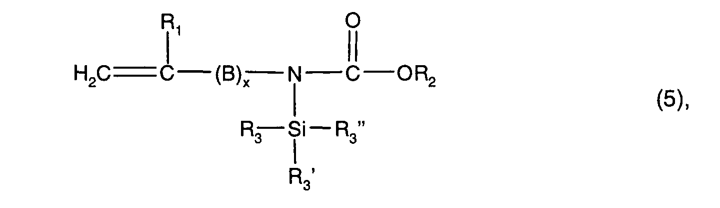 worin R₃, R₃' und R₃" unabhängig voneinander C₁-C₄-Alkyl bedeuten und die Variablen R₁, R₂, B und x die oben angegebene Bedeutung haben, überführt und dieses bei einer Temperatur von 70 bis 120°C zu einem ethylenisch ungesättigten Isocyanat der Formel (1) umsetzt.

Unter C₁-C₄-Alkyl ist dabei generell Methyl, Ethyl, n- oder iso-Propyl oder n-, iso-, sec.- oder tert.-Butyl, vorzugsweise Methyl oder Ethyl und besonders bevorzugt Methyl, zu verstehen. C₁-C₄-Alkoxy umfasst generell Methoxy, Ethoxy, n- oder iso-Propoxy oder n-, iso-, sec.- oder tert.-Butoxy, vorzugsweise Methoxy oder Ethoxy und besonders bevorzugt Methoxy.

Halogen bedeutet generell z.B. Fluor, Brom, Jod oder Chlor, vorzugsweise Brom oder Chlor und besonders bevorzugt Chlor.

R₁ bedeutet Wasserstoff oder bevorzugt Methyl.

Die Variable x steht z.B. für die Zahl 0 oder vorzugsweise für die Zahl 1.

Bei B als Phenylenrest handelt es sich z.B. um unsubstituiertes oder durch Methyl oder Methoxy substituiertes 1,2-, 1,3- oder 1,4-Phenylen. Vorzugsweise steht B als Phenylenrest für 1,3- oder 1,4-Phenylen.

Bei B als Aralkylenrest handelt es sich z.B. um unsubstituiertes oder durch Methyl oder Methoxy substituiertes Benzylen, wobei die Methylengruppe jeweils an den Isocyanatostickstoff gebunden ist. Vorzugsweise steht B als Aralkylenrest für den 1,3- oder 1,4-Phenylenmethylenrest, wobei die Methylengruppe jeweils an den Isocyanato- bzw. Urethanstickstoff gebunden ist.

Bedeutet B einen Rest der zuvor angegebenen Formel (2), so kann es sich bei B₁ z.B. um 1-Methyl- oder 1,1-Dimethylmethylen, 1,2-Ethylen, 1,2- oder 1,3-Propylen, 2-Methylpropylen oder 1,2-, 1,3-, 1,4- oder 2,3-Butylen, 2,2-Dimethyl-1,3-propylen, 2-Methyl- oder 2,3-Dimethyl-1,4-butylen, 1,2-, 1,3-, 1,4- oder 1,5-Pentylen, 2-Methyl- oder 3-Methyl- oder 4-Methylpentylen oder, 1,2-, 1,3-, 1,4-, 1,5- oder 1,6-Hexylen, 2,3-Dimethyl- oder 2,4-Dimethyl oder 3,4-Dimethyl- oder 2,3,4-Trimethyl oder 2,2,3-Trimethyl- 2,2,4-Trimethyl- oder 2,2,3,3-Tetramethyl- oder 2,2,3,4-Tetramethyl-1,5-pentylen, 2-Methyl- oder 3-Methyl oder 4-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl- oder 2,2,4-Trimethyl- oder 2,2,5-Trimethyl- oder 2,3,4-Trimethyl- oder 2,2,4,5-Tetramethyl-1,6-hexylen, 1,2-, 1,3-, 1,4- 1,5 oder 1,6-Heptylen, 2-Methyl- oder 3-Methyl- oder 4-Methyl- oder 5-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl- oder 2,2,4-Trimethyl- oder 2,2,5-Trimethyl- oder 2,2,6-Trimethyl- oder 2,3,4-Trimethyl- oder 2,4,5-Trimethyl- oder 2,4,6-Trimethyl- oder 2,2,4,5-Tetramethyl-1,7-heptylen, 1,2-, 1,3-, 1,4-1,5-1,6- oder 1,7-Octylen, 2-Methyl- oder 3-Methyl- oder 4-Methyl- oder 5-Methyl- oder 6-Methyl- oder 7-Methyl- oder 2,2-Dimethyl- oder 3,3-Dimethyl- oder 2,3-Dimethyl- oder 2,4-Dimethyl- oder 3,4-Dimethyl- oder 2,6-Dimethyl- oder 2,7-Dimethyl- oder 2,2,4-Trimethyl- oder 2,2,5-Trimethyl- oder 2,2,6-Trimethyl- oder 2,2,5,6-Tetramethyl-1,8-Octylen, 1,2-, 1,3-, 1,4- 1,5- 1,6-, 1,7- oder 1,8-Nonylen, 2-Methyl- oder 3-Methyl- oder 4-Methyl- oder 5-Methyl- oder 6-Methyl- oder 7-Methyl- oder 8-Methyl oder 2,2-Dimethyl- oder 3,3-Dimethyl- oder 2,3-Dimethyl- oder 2,4-Dimethyl- oder 3,4-Dimethyl- oder 2,6-Dimethyl- oder 2,7-Dimethyl- oder 2,8-Dimethyl- oder 2,2,4-Trimethyl- oder 2,2,5-Trimethyl- oder 2,2,6-Trimethyl- oder 2,2,7-Trimethyl- oder 2,2,8-Trimethyl-nonylen, 1,2-, 1,3-, 1,4- 1,5- 1,6-, 1,7-, 1,8- oder 1,9-Decylen, 2-Methyl- oder 3-Methyl- oder 4-Methyl- oder 5-Methyl- oder 6-Methyl- oder 7-Methyl- oder 8-Methyl- oder 9-Methyl- oder 2,2-Dimethyl- oder 3,3-Dimethyl- oder 2,3-Dimethyl- oder 2,4-Dimethyl- oder 3,4-Dimethyl- oder 2,6-Dimethyl- oder 2,7-Dimethyl-, 2,8-Dimethyl- oder 2,9-Dimethyl-1,10-decylen, 1,2-, 1,3-, 1,4- 1,5- 1,6-, 1,7-, 1,8-, 1,9- oder 1,10-Undecylen, 2-Methyl- oder 3-Methyl- oder 4-Methyl- oder 5-Methyl- oder 6-Methyl- oder 7-Methyl- oder 8-Methyl- oder 9-Methyl- oder 10-Methyl-1,11-undecylen, 1,4- 1,5- 1,6-, 1,7-, 1,8-, 1,9-, 1,10- oder 1,11-Dodecylen handeln.

Beispiele für durch Sauerstoffatome unterbrochenes Alkylen sind -CH₂CH₂-O-CH₂CH₂-, -CH₂CH₂-O-CH₂CH₂-O-CH₂CH₂-, -CH₂CH₂-O-CH₂CH₂-O-CH₂CH₂-O-CH₂CH₂-, [-CH(CH₃)CH₂-O-CH(CH₃)CH₂-], -CH(CH₃)CH₂-O-CH₂CH₂-, -CH(C₂H₅)CH₂-O-CH₂CH₂-, [-CH(C₂H₅)CH₂-O-CH(C₂H₅)CH₂-] oder -CH₂CH₂CH₂CH₂-O-CH₂CH₂CH₂CH₂-.

B₁ bedeutet bevorzugt lineares oder verzweigtes C₂-C₈-Alkylen, besonders bevorzugt lineares C₂-C₈-Alkylen und besonders bevorzugt lineares C₂-C₄-Alkylen. In einer bevorzugten Ausführungsform der Erfindung bedeutet B₁ 1,2-Ethylen.

B bedeutet bevorzugt einen Rest der zuvor angegebenen Formel (2), worin für die darin enthaltene Variable B₁ die zuvor genannten Bedeutungen und Bevorzugungen gelten.

R₂ steht bevorzugt für C₁-C₂-Alkyl oder für unsubstituiertes oder durch Methyl, Methoxy oder Chlor substituiertes Phenyl und besonders bevorzugt für Methyl, Ethyl oder Phenyl. Beispiele für insbesonders bevorzugte Reste R₂ sind Ethyl oder noch bevorzugter Phenyl.

Für die Silylierung der ungesättigten Urethanvorläufer kommen prinzipiell alle üblicherweise verwendeten Silylierungsmittel in Frage, wie sie z.B. aus A.E. Pierce, Silylation of Organic Compounds, Pierce Chemical Corp. Rockford III. 1968, Seiten 7-26, bekannt sind. Beispiele für geeignete Silylierungsmittel sind Alkylsilylhalogenide, Alkylsilylamine, Hexaalkyl-disilazane oder N-Silylacetamide.

Das Silylierungsmittel entspricht z.B. der Formel 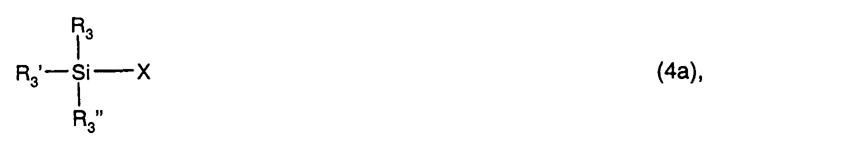 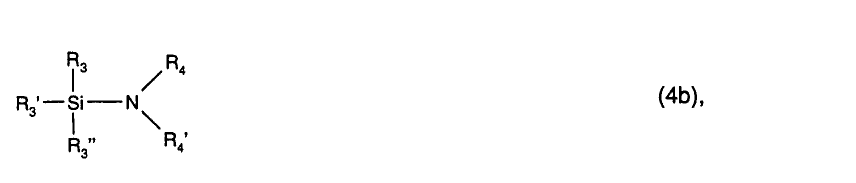 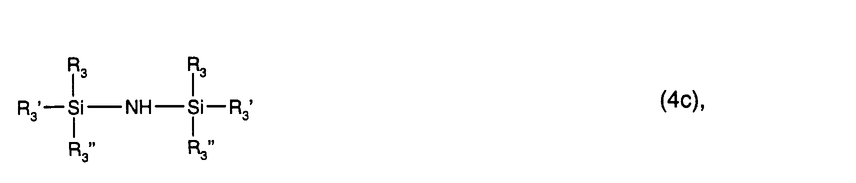 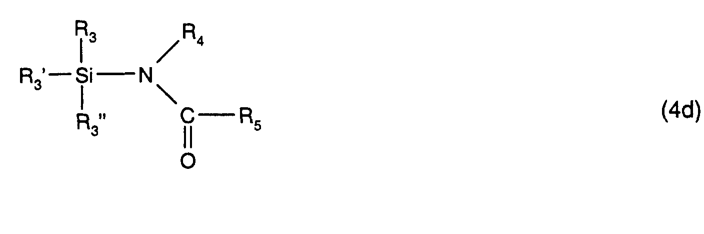 oder 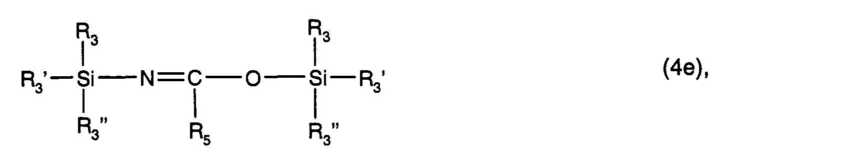 worin X Halogen, z.B. Brom oder Chlor und vorzugsweise Chlor, ist, R₃, R₃' und R₃" unabhängig voneinander je C₁-C₄-Alkyl, bevorzugt Methyl oder Ethyl und insbesondere bevorzugt je Methyl bedeuten, R₄ und R₄' unabhängig voneinander je Wasserstoff oder C₁-C₄-Alkyl und vorzugsweise Wasserstoff, Methyl oder Ethyl sind, und R₅ für C₁-C₄-Alkyl, vorzugsweise für Methyl oder Ethyl und insbesondere bevorzugt für Methyl, steht. In den Formeln (4a) - (4e) sind die Variablen R₃, R₃' und R₃" verschieden oder vorzugsweise identisch.

Beispiele für bevorzugte Silylierungsmittel sind Trimethyl- oder Triethyllsilylbromid, Trimethyl- oder Triethylsilylchlorid, Hexamethyldisilazan, N-Trimethylsilyl-N,N-diethylamin, N-Trimethylsilyl-N-methylamin, N,O-Bis-(trimethylsilyl)acetamid oder N-Trimethylsilyl-N-methylacetamid. Besonders bevorzugt als Silylierungsmittel sind Trimethylsilylchlorid, Hexamethyldisilazan, N-Trimethylsilyl-N,N-diethylamin, N,O-Bis-(trimethylsilyl)acetamid oder N-Trimethylsilyl-N-methylacetamid. Möglich sind auch Gemische mehrerer verschiedener Silylierungsmittel, z.B. Trimethylsilylchlorid/ Hexamethyldisilazan.

Die Silylierung der ethylenisch ungesättigten Urethan-Vorläufer z.B. der Formel (3) findet vorteilhaft in einem aprotischen organischen Lösungsmittel statt, wobei sowohl unpolare als auch dipolare Lösungsmittel in Frage kommen. Beispiele für geeignete Lösungsmittel sind aliphatische oder aromatische Kohlenwasserstoffe wie z.B. Benzol, Toluol, Xylol oder Xylolgemische oder Cyclohexan, halogenierte Kohlenwasserstoffe wie z.B. Methylenchlorid oder Chloroform, Ether wie Dioxan oder Tetrahydrofuran, sowie dipolar-aprotische Lösungsmittel wie z.B. Formamid, N,N-Dimethylformamid, Pyridin oder Acetonitril. Bevorzugt als organische Lösungsmittel sind aromatische oder aliphatische Kohlenwasserstoffe wie z.B. Benzol Toluol, Xylol oder Xylolgemische oder Cyclohexan oder cyclische Ether wie insbesondere Dioxan oder Tetrahydrofuran. Besonders bevorzugte Lösungsmittel sind Benzol, Xylol oder Xylolgemische oder insbesondere Toluol. Möglich sind auch Gemische verschiedener Lösungsmittel. Es erweist sich darüberhinaus oft als günstig, dem Reaktionsgemisch einen nicht-flüchtigen Polymerisationsinhibitor, z.B. Di-tert.-butyl-p-Kresol, zuzusetzen.

Die bei der Silylierung verwendete Temperatur hängt in erster Linie von der Reaktivität des Silylierungsmittels und vom verwendeten Lösungsmittel ab und kann daher innerhalb weiter Grenzen schwanken. Vorteilhaft haben sich Temperaturen von 0 bis 60°C oder vorzugsweise von ca. 15 bis ca. 50°C erwiesen. Die Reaktionszeiten können ebenfalls innerhalb weiter Grenzen, z.B. zwischen ca. 15 Minuten und 24 Stunden, schwanken.

Je nach dem verwendeten Silylierungsmittel kann es von Vorteil sein, dem Silylierungsgemisch einen Säureakzeptor oder einen Säurekatalysator zuzusetzen. Der Zusatz von Säureakzeptoren ist bevorzugt bei der Verwendung von Verbindungen der zuvor angegebenen Formel (4a), z.B. von Trimethylchlorsilan, als Silylierungsmittel. Als Säureakzeptoren kommen im Prinzip alle basisch wirkende Verbindungen in Frage. Beispiele sind organische, insbesondere aliphatische oder aromatische, Amine, z.B. Pyridin oder Tri-C₁-C₄-alkylamine und bevorzugt Triethylamin. Der Zusatz von Säurekatalysatoren ist bevorzugt bei der Verwendung von Verbindungen der zuvor angegebenen Formeln (4b) oder (4c). Als Säurekatalysator kann z.B. eine Verbindung der oben angegebenen Formel (4a), z.B. Trimethylchlorsilan, oder eine organische Säure, z.B. Trichloressigsäure, dienen.

Das Silylierungsmittel wird in mindestens äquimolaren Mengen oder vorzugsweise in einem z.B. bis zu 100 Mol-% betragenden molaren Ueberschuss, bezogen auf das zu silylierende Urethan, eingesetzt. Ein vorhandener molarer Ueberschuss des Silylierungsmittel beträgt vorzugsweise 5-50 Mol-% und insbesondere bevorzugt 5-30 Mol-%, jeweils bezogen auf das zu silylierende Urethan. Ein gegebenenfalls anwesender Säureakzeptor ist vorteilhaft in einer in etwa äquimolaren Menge, bezogen auf das Silylierungsmittel, anwesend.

Die gebildeten ethylenisch ungesättigten N-Silylurethane, die z.B. der zuvor angegebenen Formel (5) entsprechen, können isoliert und gereinigt oder aber direkt ohne Aufarbeitung thermisch unter Abspaltung eines Silylethers in die gewünschten Isocyanate überführt werden. Die Thermolyse findet vorteilhaft in einem inerten Lösungsmittel, z.B. in einem der zuvor genannten aliphatischen oder aromatischen Kohlenwasserstoffe, bevorzugt in einem aromatischen Kohlenwasserstoff wie Benzol, Toluol oder Xylol und besonders bevorzugt in Toluol, bei einer Temperatur von 70 bis 120°C und besonders bevorzugt bei 80 bis 100°C, statt. Es erweist sich darüberhinaus als günstig, die Umsetzung und/oder Aufarbeitung in Gegenwart eines nicht-flüchtigen Polymerisationsinhibitors, z.B. Di-tert.-butyl-p-kresol, durchzuführen.

Die erhaltenen Verbindungen der Formel (1) können in an sich bekannter Weise, z.B. durch Destillation, aus dem Reaktionsgemisch isoliert und gereinigt werden. Der Fachmann ist sich bewusst, dass die erfindungsgemässen Reaktionsschritte und die Aufarbeitung der Reaktionsgemische wegen der Feuchtigkeitsempfindlichkeit der Endprodukte und der silylierten Zwischenprodukte im allgemeinen unter Schutzgas, z.B unter Stickstoff oder Argon, durchgeführt werden.

Eine bevorzugte Ausführungsform der vorliegenden Erfindung betrifft ein Verfahren zur Herstellung von Verbindungen der zuvor angegebenen Formel (1), worin x die Zahl 1 bedeutet, B für einen Rest der Formel (2), worin B₁ lineares oder verzweigtes C₂-C₈-Alkylen ist, steht und R₁ Wasserstoff oder Methyl bedeutet, dadurch gekennzeichnet, dass man eine Verbindung der zuvor angegebenen Formel (3), worin R₂ Ethyl oder Phenyl bedeutet und R₁, B und x die oben angegebene Bedeutung haben, mit einem Silylierungsmittel ausgewählt aus der Gruppe Alkylsilylhalogenide, Alkylsilylamine, Hexaalkyl-disilazane und N-Silylacetamide zu einer Verbindung der zuvor angegebenen Formel (5) umsetzt und diese direkt ohne Aufarbeitung einer Thermolyse bei einer Temperatur von 70 bis 120°C unterwirft.

Die Urethan-Vorläufer, die z.B. der zuvor angegebenen Formel (3) entsprechen, sind an sich bekannt oder können in an sich bekannter Weise erhalten werden.

Die Vorprodukte der zuvor angegebenen Formel (3), worin x für die Zahl 0 steht, können z.B. erhalten werden, indem man ein Vinylhalogenid, z.B. Vinylbromid, mit einem Carbamidsäureester, z.B. Urethan, umsetzt.

Verbindungen der Formel (3), worin x für die Zahl 1 steht, sind z.B. zugänglich, indem man eine Verbindung der Formel 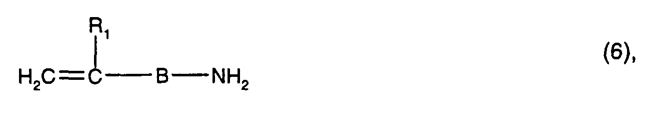 worin B und R₁ jeweils die zuvor angegebene Bedeutung haben, mit einem Halogenameisensäureester der Formel 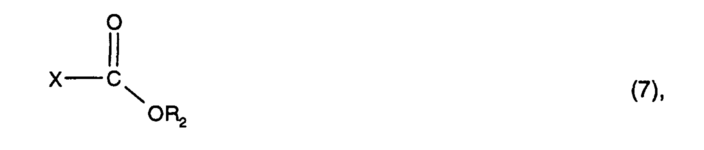 worin X Halogen, bevorzugt Chlor, ist und R₂ die zuvor angegebene Bedeutung hat, umsetzt.

Die Synthese von Verbindungen der Formel (3), worin B ein Rest der zuvor angegebenen Formel (2) ist, kann vorteilhaft auch erfolgen, indem man eine Verbindung der Formel 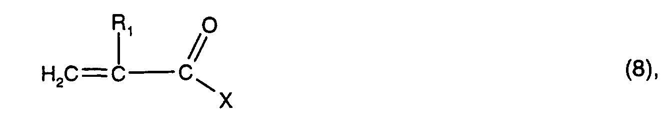 worin X Halogen, bevorzugt Chlor, ist und R₁ die zuvor angegebene Bedeutung hat, mit einer Verbindung der Formel 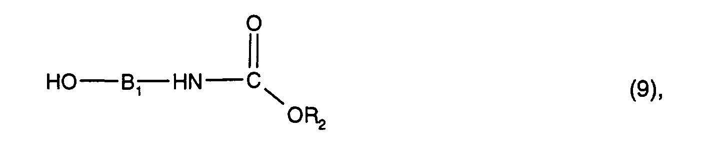 worin B₁ und R₂ jeweils die zuvor angegebene Bedeutung haben, umsetzt. Die Verbindungen der Formeln (6), (7), (8) und (9) sind allesamt bekannt oder können nach an sich bekannten Methoden hergestellt werden.

Eine bevorzugte Ausführungsform der vorliegenden Erfindung betrifft ein Verfahren zur Herstellung von Verbindungen der Formel 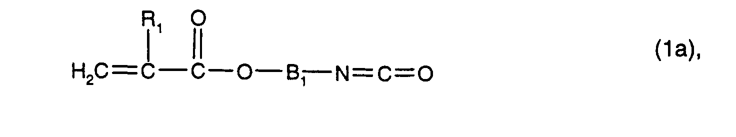 worin R₁ Wasserstoff oder Methyl ist und B₁ lineares oder verzweigtes C₂-C₁₂-Alkylen, welches gegebenenfalls durch ein oder mehrere Sauerstoffatome unterbrochen ist, bedeutet, dadurch gekennzeichnet, dass man
(a) eine Verbindung der Formel 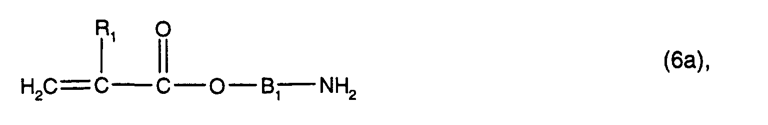 oder vorzugsweise ein Säureadditionssalz, z.B. ein Hydrohalogenid wie insbesondere das Hydrochlorid davon, worin R₁ und B₁ jeweils die zuvor angegebene Bedeutung haben, mit einem Halogenameisensäureester der Formel 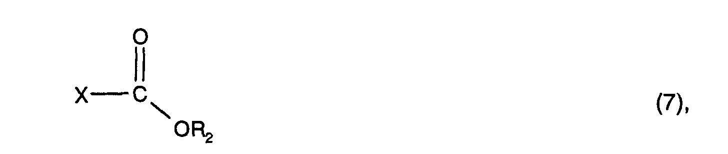 worin X Halogen und R₂ C₁-C₄-Alkyl oder gegebenenfalls durch C₁-C₄-Alkyl, C₁-C₄-Alkoxy oder Halogen substituiertes Phenyl sind, zu einer Verbindung der Formel 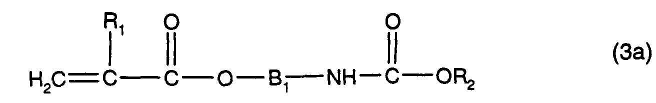 umsetzt,
(b) die gemäss (a) erhältliche Urethanverbindung mit einem Silylierungsmittel in eine Verbindung der Formel 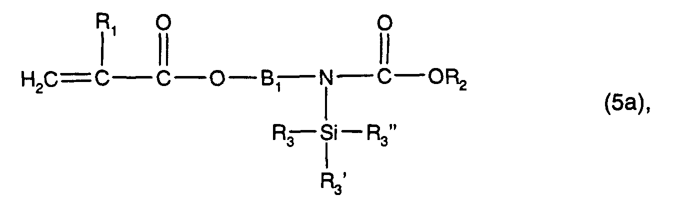 worin R₃, R₃' und R₃" unabhängig voneinander C₁-C₄-Alkyl bedeuten und die Variablen R₁, R₂ und B₁ oben angegebene Bedeutung haben, überführt und
(c) die gemäss (b) erhältliche N-Silylurethanverbindung mittels Thermolyse bei einer Temperatur von 70 bis 120°C zu einer Verbindung der Formel (1a) umsetzt.

Eine weitere bevorzugte Ausführungsform der vorliegenden Erfindung betrifft ein Verfahren zur Herstellung von Verbindungen der Formel 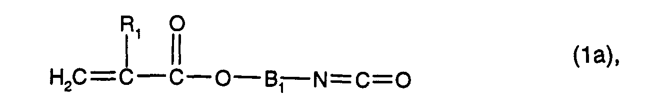 worin R₁ Wasserstoff oder Methyl ist und B₁ lineares oder verzweigtes C₂-C₁₂-Alkylen, welches gegebenenfalls durch ein oder mehrere Sauerstoffatome unterbrochen ist, bedeutet, dadurch gekennzeichnet, dass man
(a) eine Verbindung der Formel 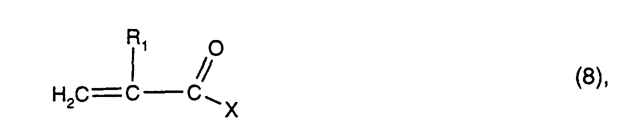 worin R₁ die zuvor angegebene Bedeutung hat und X Halogen ist, mit einer Verbindung der Formel 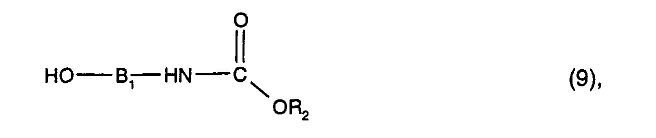 worin R₂ C₁-C₄-Alkyl oder gegebenenfalls durch C₁-C₄-Alkyl, C₁-C₄-Alkoxy oder Halogen substituiertes Phenyl ist und B₁ die oben angegebene Bedeutung hat, zu einer Verbindung der Formel 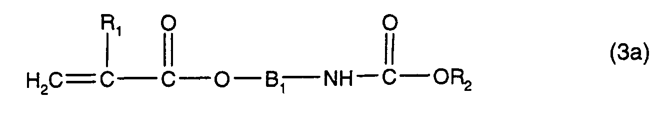 umsetzt,
(b) die gemäss (a) erhältliche Urethanverbindung mit einem Silylierungsmittel in eine Verbindung der Formel 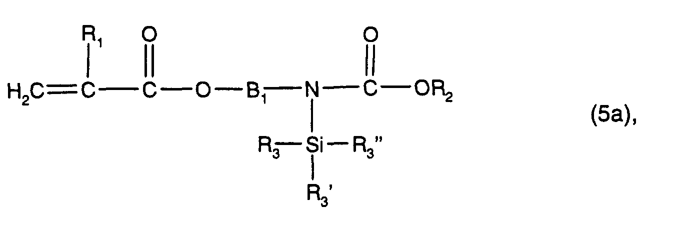 worin R₃, R₃' und R₃" unabhängig voneinander C₁-C₄-Alkyl bedeuten und die Variablen R₁, R₂ und B₁ oben angegebene Bedeutung haben, überführt und
(c) die gemäss (b) erhältliche N-Silylurethanverbindung mittels Thermolyse bei einer Temperatur von 70 bis 120°C zu einer Verbindung der Formel (1a) umsetzt.

Die nach dem erfindungsgemässen Verfahren herstellbaren ethylenisch ungesättigten Isocyanate, die z.B. der zuvor angegebenen Formel (1) entsprechen können, können als Ausgangsprodukte für die Herstellung von verschiedensten Produkten z.B. im Bereich von Pharmazeutika oder Insektiziden dienen oder finden vorzugsweise als Monomere oder Comonomere für die Herstellung von Polymerisaten oder Copolymerisaten Verwendung.

Die Verbindungen sind von besonderem Nutzen für die Synthese von polymerisierbaren Makromeren und Makromonomeren, wie sie für die Herstellung von zahlreichen biomedizinischen Artikeln und Werkstoffen, z.B. Kontaktlinsen, Verwendung finden.

Die nachfolgenden Beispiele dienen der weiteren Illustration der vorliegenden Erfindung; sie sollen diese jedoch in ihrem Umfang in keiner Weise einschränken. Temperaturen sind, wenn nicht anders vermerkt, in Grad Celsius angegeben.

### Herstellung der Urethane der Formel (3)

Beispiel 1: 608 ml Ethanolamin werden in einem Reaktionsgefäss unter Stickstoffatmosphäre in 13 Litern trockenem Methylenchlorid gelöst und anschliessend 6,7 g Tetrabutylammoniumbromid und 2378 g Natriumcarbonat ergänzt. Das Gemisch wird bis zur Bildung einer homogenen Suspension gerührt und auf 10°C abgekühlt. Danach wird ein Gemisch von 1402 ml Chlorameisensäurephenylester in 5 Litern trockenem Methylenchlorid innerhalb von ca. 11 Stunden tropfenweise bei einer Temperatur von 10-15°C zugetropft, und die einsetzende exotherme Reaktion durch Aussenkühlung mit Eis kontrolliert. Nach beendeter Zugabe wird noch bis zur vollständigen Umsetzung der Edukte weitergerührt (ca. 30 Minuten bei 25°C). Die erhaltene feine Suspension wird filtriert und dann mittels eines Rotationsverdampfers auf ein Volumen von ca. 1 Liter eingeengt. Man ergänzt 5 Liter n-Hexan, rührt die erhaltene Suspension über Nacht bei Raumtemperatur (25°C), kühlt dann auf 10°C ab und filtriert den Feststoff ab. Das erhaltene kristalline Produkt wird mit zwei 500ml-Portionen n-Hexan gewaschen und im Vakuum bei 30°C getrocknet. Man erhält die Verbindung N-(2-Hydroxyethyl)-phenylcarbamat als weisses kristallines Produkt vom Schmelzpunkt 79-80°C.

620,3 g des oben hergestellten N-(2-Hydroxyethyl)-phenylcarbamats und 7,5 Liter Methylenchlorid werden unter Stickstoffatmosphäre in einem Reaktionsgefäss vereinigt. Zur erhaltenen Suspension werden 1000 ml frisch destilliertes Methacryloylchlorid gegeben. Man erwärmt über Nacht auf 80°C und leitet dabei einen sanften Argon-Schutzgasstrom durch das Reaktionsgefäss. Es bildet sich eine klare Lösung, welche auf 25°C abgekühlt und mittels Rotationsverdampfer vollständig vom Lösungsmittel befreit wird. Der erhaltene kristalline Feststoff wird aus einem Gemisch von 5 Litern Toluol und 3 Litern n-Hexan umkristallisiert, anschliessend mit 500 ml n-Hexan gewaschen und im Vakuum bei 30°C getrocknet. Man erhält die Verbindung N-(2-Methacryloyloxyethyl)-phenylcarbamat als weisses kristallines Produkt vom Schmelzpunkt 105-106°C.

Beispiel 2: In einem unter trockenem Stickstoff ausgeheizten Reaktionsgefäss werden unter Stickstoffatmosphäre 15,56 g 2-Aminoethylmethacrylat Hydrochlorid in 30 ml trockenem N,N-Dimethylacetamid gelöst und die klare, hellgelbe Lösung auf ca. 4°C abgekühlt. Anschliessend werden bei dieser Temperatur simultan (a) eine Lösung von 15,66 g Chlorameisensäurephenylester in 50 ml trockenem Methylenchlorid und (b) eine Lösung von 20,5 g Triethylamin in 50 ml trockenem Methylenchlorid zugetropft. Nach 1,5 Stunden bei ca. 4°C wird auf Raumtemperatur erwärmt und noch ca.12 Stunden weitergerührt. Man setzt 15 mg Di-tert.-butyl-p-kresol zu und befreit die erhaltene weisse Suspension am Rotationsverdampfer von der Hauptmenge Lösungsmittel. Die unter Zusatz von 200 ml Methylenchlorid verdünnte Suspension wird dreimal mit je 200 ml Wasser extrahiert und anschliessend die organische Phase mit Natriumsulfat getrocknet. Nach dem Eindampfen am Rotationsverdampfer wird der erhaltene Rückstand aus Diethylether/Methylenchlorid im Verhältnis 4:1 umkristallisiert. Es wird die Verbindung N-(2-Methacryloyloxyethyl)-phenylcarbamat als weisses kristallines Produkt vom Schmelzpunkt 110°C erhalten.

Beispiel 3: Verfährt man wie im Beispiel 1 beschrieben und setzt 18,1 g N-(2-Hydroxyethyl)-phenylcarbamat in trockenem Methylenchlorid mit 9,05 g Acryloylchlorid und 10,1 g Triethylamin um, erhält man die Verbindung N-(2-Acryloyloxyethyl)-phenylcarbamat, welche aus Toluol/Hexan umkristallisiert wird.

### Herstellung der Isocyanate der Formel (1)

Beispiel 4: 400 g des gemäss Beispiel 1 oder 2 erhaltenen N-(2-Methacryloyloxyethyl)phenylcarbamats werden unter Argon-Schutzgas bei strengem Feuchtigkeitsausschluss in 3 Litern trockenem Toluol suspendiert. Nach Zugabe von 240 g trockenem Triethyamin und 4g Di-tert.-butyl-p-kresol wird das Reaktionsgemisch auf 50°C erwärmt und 204 g frisch destilliertes Trimethylsilylchlorid tropfenweise über einen Zeitraum von ca. 20 Minuten zugegeben. Nach 6 Stunden bei 50°C ist die Silylierung vollständig, und es hat sich eine feine Triethylammoniumchlorid enthaltende Suspension gebildet.

Die thermische Eliminierung des Phenoxy-trimethylsilans aus dem erhaltenen N-silylierten Urethan wird ausgeführt, indem man die Suspension ca. 8 Stunden lang unter Rückfluss (ca. 100°C) erhitzt. Anschliessend lässt man über Nacht bei Raumtemperatur rühren, um das Triethylammoniumchlorid vollständig zur Kristallisation zu bringen. Zur Aufarbeitung des Reaktionsgemisches wird eine Lösung von 2g Di-tert.-butyl-p-kresol in 100 ml trockenem Toluol zugegeben und anschliessend niedrig-siedende Komponenten (überschüssiges Triethylamin, Trimethylsilylchlorid) mittels Destillation bei ca. 80-100°C entfernt. Nach dem Abkühlen auf 25°C wird das Triethylammoniumchlorid aus der Reaktionssuspension unter Argon-Schutzgasatmosphäre abfiltriert, mehrfach mit trockenem Toluol gewaschen und die gesammelten Filtrate am Rotationsverdampfer auf eine Menge von ca. 680 g eingeengt. Dieser Rest wird zunächst schnell ohne sorgfältige Fraktionierung bei ca. 4-5 mbar destilliert. Die Fraktionen, die einen Siedepunkt zwischen 60 und 80°C aufweisen, werden gesammelt und nach Zugabe von weiteren 2g Di-tert.-butyl-p-kresol einer Fraktionierung mittels Vigreux-Kolonne unterworfen. Man erhält die Verbindung 2-lsocyanatoethyl-methacrylat als farbloses Oel, welches einen Siedepunkt von 60°C bei 7 mbar aufweist.

Beispiel 5: Analog wie im Beispiel 4 beschrieben werden 47,6 g des gemäss Beispiel 3 hergestellten N-(2-Acryloyloxyethyl)-phenylcarbamats in trockenem Toluol mit 22,8 g Trimethylchlorsilan und 21,2 g Triethylamin silyliert. Man verfährt ohne Isolierung des Zwischenprodukts weiter und erhält nach der Thermolyse und einer anschliessenden fraktionierten Destillation die Verbindung 2-lsocyanatoethylacrylat, welche einen Siedepunkt von 52-54°C bei 5 mbar aufweist.

## Patentansprüche

1. Verfahren zur Herstellung von ethylenisch ungesättigten Isocyanaten der Formel 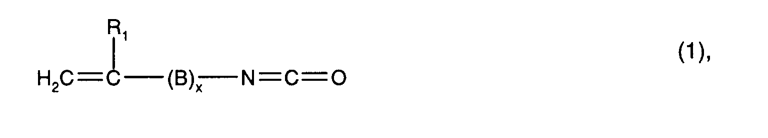 worin
R₁ für Wasserstoff oder Methyl steht,
x die Zahl 0 oder 1 bedeutet,
B gegebenenfalls durch C₁-C₄-Alkyl oder C₁-C₄-Alkoxy substituiertes Phenylen oder C₇-C₁₂-Aralkylen oder ein Rest der Formel 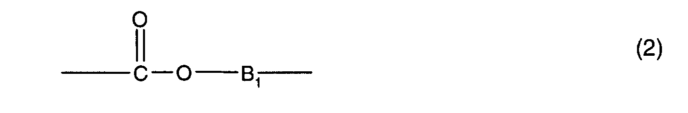 ist und B₁ lineares oder verzweigtes C₂-C₁₂-Alkylen, welches gegebenenfalls durch ein oder mehrere Sauerstoffatome unterbrochen ist, bedeutet, **dadurch gekennzeichnet, dass** man ein ethylenisch ungesättigtes Urethan der Formel 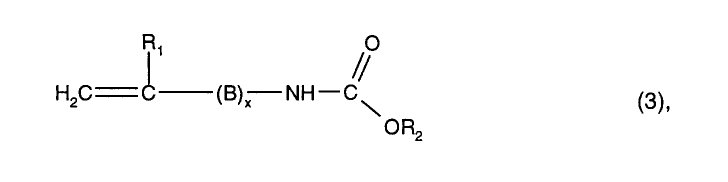 worin R₂ C₁-C₄-Alkyl oder gegebenenfalls durch C₁-C₄-Alkyl, C₁-C₄-Alkoxy oder Halogen substituiertes Phenyl ist und die Variablen R₁, B und x die oben angegebene Bedeutung haben, mit einem Silylierungsmittel in ein ethylenisch ungesättigtes N-Silyl-Urethan der Formel 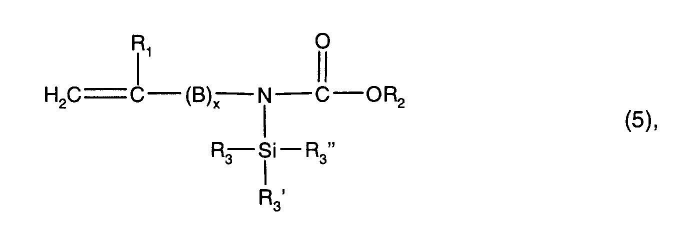 worin R₃, R₃' und R₃" unabhängig voneinander C₁-C₄-Alkyl bedeuten und die Variablen R₁, R₂, B und x die oben angegebene Bedeutung haben, überführt und dieses bei einer Temperatur von 70 bis 120°C zu einem ethylenisch ungesättigten Isocyanat der Formel (1) umsetzt.

2. Verfahren gemäss Anspruch 1, **dadurch gekennzeichnet, dass** x für die Zahl 1 steht.

3. Verfahren gemäss Anspruch 1 oder 2, **dadurch gekennzeichnet, dass** x für die Zahl 1 steht und B einen Rest der im Anspruch 1 angegebenen Formel (2) bedeutet.

4. Verfahren gemäss einem der Ansprüche 1 bis 3, **dadurch gekennzeichnet, dass** B₁ lineares oder verzweigtes C₂-C₈-Alkylen, vorzugsweise lineares C₂-C₄-Alkylen, bedeutet.

5. Verfahren gemäss einem der Ansprüche 1 bis 4, **dadurch gekennzeichnet, dass** R₂ C₁-C₂-Alkyl oder unsubstituiertes oder durch Methyl, Methoxy oder Chlor substituiertes Phenyl, vorzugsweise Ethyl oder Phenyl, bedeutet.

6. Verfahren gemäss einem der Ansprüche 1 bis 5, **dadurch gekennzeichnet, dass** es sich bei dem Silylierungsmittel um ein Alkylsilylhalogenid, Alkylsilylamin, Hexaalkyl-disilazan oder N-Silylacetamid handelt.

7. Verfahren gemäss einem der Ansprüche 1 bis 6, **dadurch gekennzeichnet, dass** das Silylierungsmittel der Formel 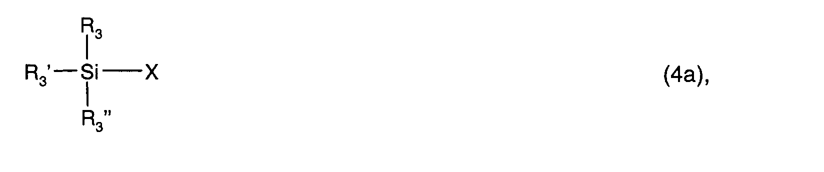 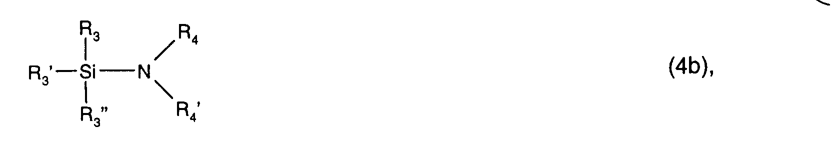 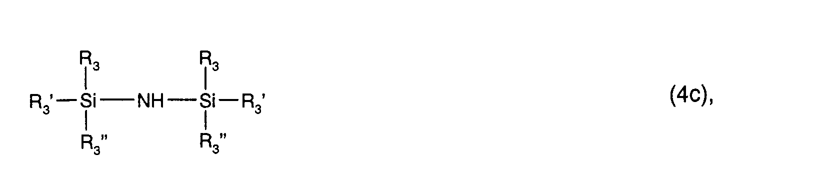 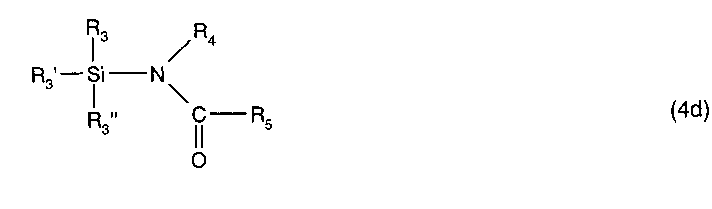 oder 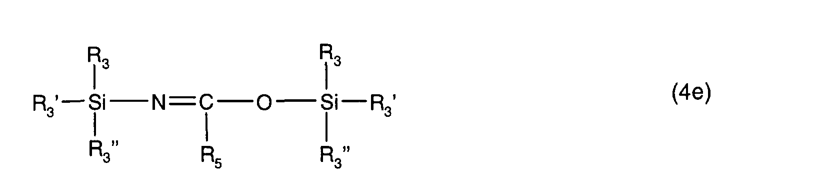 entspricht, worin X Halogen ist, R₃, R₃' und R₃" unabhängig voneinander je C₁-C₄-Alkyl, bedeuten, R₄ und R₄' unabhängig voneinander je Wasserstoff oder C₁-C₄-Alkyl sind, und R₅ für C₁-C₄-Alkyl steht.

8. Verfahren gemäss einem der Ansprüche 1 bis 7, **dadurch gekennzeichnet, dass** es sich bei dem Silylierungsmittel um Trimethylsilylchlorid, Hexamethyldisilazan, N-Trimethylsilyl-N,N-diethylamin, N,O-Bis-(trimethylsilyl)acetamid oder N-Trimethylsilyl-N-methylacetamid oder um ein Gemisch aus Trimethylsilylchlorid und Hexamethyldisilazan handelt.

9. Verfahren gemäss einem der Ansprüche 1 bis 8, **dadurch gekennzeichnet, dass** man die Silylierung des ethylenisch ungesättigten Urethans unter Schutzgasatmosphäre in einem aprotischen organischen Lösungsmittel bei einer Temperatur von 15 bis 50°C durchführt.

10. Verfahren gemäss einem der Ansprüche 1 bis 9, **dadurch gekennzeichnet, dass** man das ethylenisch ungesättigte N-Silyl-Urethan direkt ohne Aufarbeitung thermisch in das ethylenisch ungesättigte Isocyanat umwandelt.

11. Verfahren gemäss einem der Ansprüche 1 bis 10, **dadurch gekennzeichnet, dass** man die Thermolyse des ethylenisch ungesättigten N-Silyl-Urethans zum ethylenisch ungesättigten Isocyanat unter Schutzgasatmosphäre in einem aprotischen Lösungsmittel durchführt.

12. Verfahren gemäss Anspruch 1 zur Herstellung von Verbindungen der Formel (1), worin x die Zahl 1 bedeutet, B für einen Rest der Formel (2), worin B₁ lineares oder verzweigtes C₂-C₈-Alkylen ist, steht und R₁ Wasserstoff oder Methyl bedeutet, **dadurch gekennzeichnet, dass** man eine Verbindung der Formel (3), worin R₂ Ethyl oder Phenyl bedeutet und R₁, B und x die oben angegebene Bedeutung haben, mit einem Silylierungsmittel ausgewählt aus der Gruppe Alkylsilylhalogenide, Alkylsilylamine, Hexaalkyl-disilazane und N-Silylacetamide zu einer Verbindung der Formel (5) umsetzt und diese direkt ohne Aufarbeitung einer Thermolyse bei einer Temperatur von 70 bis 120°C unterwirft.

13. Verfahren gemäss Anspruch 12, **dadurch gekennzeichnet, dass** R₂ Phenyl ist und die Thermolyse bei einer Temperatur von 80 bis 100°C stattfindet.

14. Verfahren zur Herstellung von Verbindungen der Formel 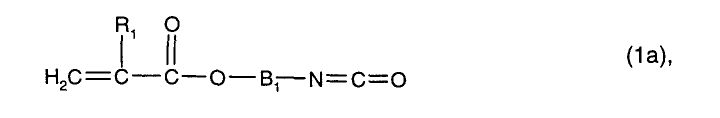 worin R₁ Wasserstoff oder Methyl ist und B₁ lineares oder verzweigtes C₂-C₁₂-Alkylen, welches gegebenenfalls durch ein oder mehrere Sauerstoffatome unterbrochen ist, bedeutet, **dadurch gekennzeichnet, dass** man
(a) eine Verbindung der Formel 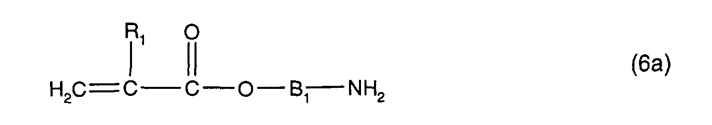 oder ein Säureadditionssalz davon, worin R₁ und B₁ jeweils die zuvor angegebene Bedeutung haben, mit einem Halogenameisensäureester der Formel 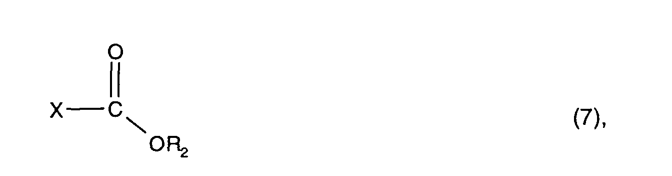 worin X Halogen und R₂ C₁-C₄-Alkyl oder gegebenenfalls durch C₁-C₄-Alkyl, C₁-C₄-Alkoxy oder Halogen substituiertes Phenyl sind, zu einer Verbindung der Formel 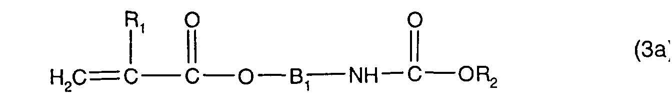 umsetzt,
(b) die gemäss (a) erhältliche Urethanverbindung mit einem Silylierungsmittel in eine Verbindung der Formel 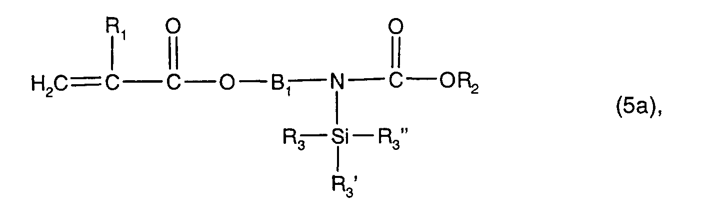 worin R₃, R₃' und R₃" unabhängig voneinander C₁-C₄-Alkyl bedeuten und die Variablen R₁, R₂ und B₁ oben angegebene Bedeutung haben, überführt und
(c) die gemäss (b) erhältliche N-Silylurethanverbindung mittels Thermolyse bei einer Temperatur von 70 bis 120°C zu einer Verbindung der Formel (1a) umsetzt.

15. Verfahren zur Herstellung von Verbindungen der Formel 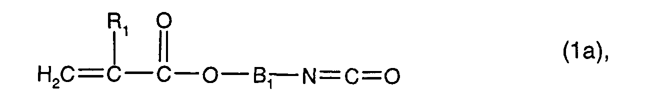 worin R₁ Wasserstoff oder Methyl ist und B₁ lineares oder verzweigtes C₂-C₁₂-Alkylen, welches gegebenenfalls durch ein oder mehrere Sauerstoffatome unterbrochen ist, bedeutet, **dadurch gekennzeichnet, dass** man
(a) eine Verbindung der Formel 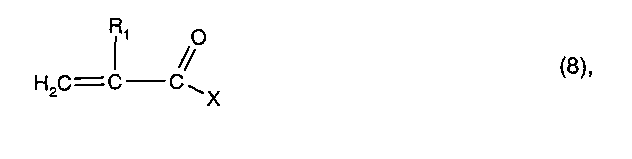 worin R₁ die zuvor angegebene Bedeutung hat und X Halogen ist, mit einer Verbindung der Formel 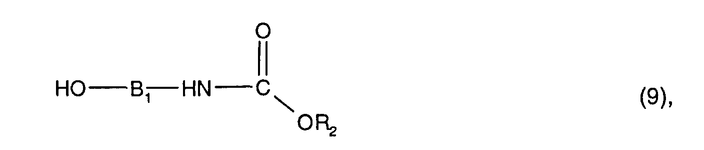 worin R₂ C₁-C₄-Alkyl oder gegebenenfalls durch C₁-C₄-Alkyl, C₁-C₄-Alkoxy oder Halogen substituiertes Phenyl ist und B₁ die oben angegebene Bedeutung hat, zu einer Verbindung der Formel 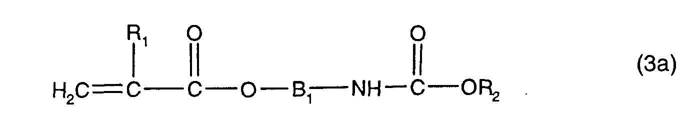 umsetzt,
(b) die gemäss (a) erhältliche Urethanverbindung mit einem Silylierungsmittel in eine Verbindung der Formel 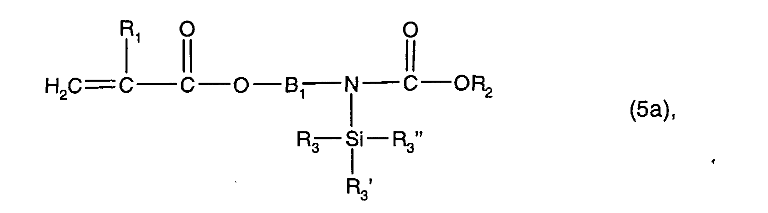 worin R₃, R₃' und R₃" unabhängig voneinander C₁-C₄-Alkyl bedeuten und die Variablen R₁, R₂ und B₁ oben angegebene Bedeutung haben, überführt und
(c) die gemäss (b) erhältliche N-Silylurethanverbindung mittels Thermolyse bei einer Temperatur von 70 bis 120°C zu einer Verbindung der Formel (1a) umsetzt.

## Claims

1. A process for the preparation of ethylenically unsaturated isocyanates of formula 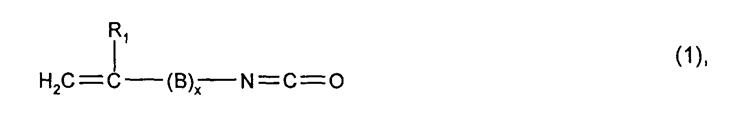 wherein
R₁ is hydrogen or methyl,
x is the number 0 or 1,
B is phenylene or C₇-C₁₂aralkylene each of which is unsubstituted or substituted by C₁-C₄alkyl or by C₁-C₄alkoxy or is a radical of formula 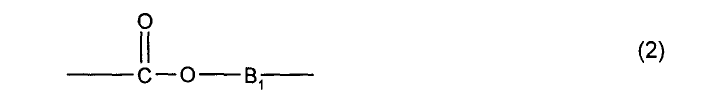 and B₁ is linear or branched C₂-C₁₂alkylene that is uninterrupted or is interrupted by one or more oxygen atoms, which process comprises converting an ethylenically unsaturated urethane of formula 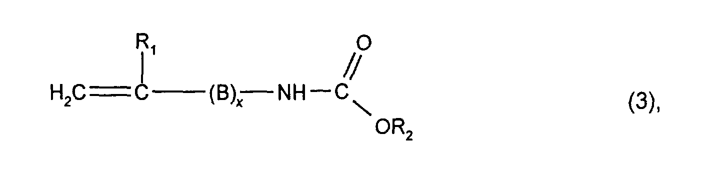 wherein R₂ is C₁-C₄alkyl or is phenyl that is unsubstituted or substituted by C₁-C₄alkyl, C₁-C₄alkoxy or by halogen and the variables R₁, B and x are as defined above, with the aid of a silylating agent, into an ethylenically unsaturated N-silyl-urethane of formula 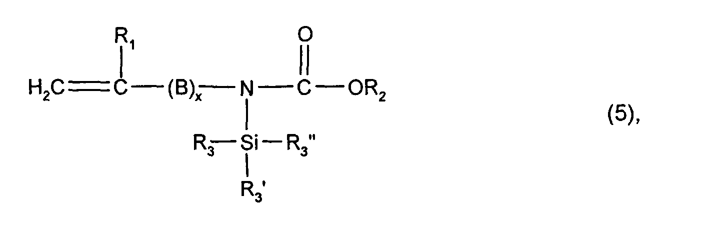 wherein R₃, R₃' and R₃" are each independently of the others C₁-C₄alkyl and the variables R₁, R₂, B and x are as defined above, and converting that compound at a temperature of from 70 to 120°C into an ethylenically unsaturated isocyanate of formula (1).

2. A process according to claim 1, wherein x is the number 1.

3. A process according to either claim 1 or claim 2, wherein x is the number 1 and B is a radical of formula (2) indicated in claim 1.

4. A process according to any one of claims 1 to 3, wherein B₁ is linear or branched C₂-C₈alkylene, preferably linear C₂-C₄alkylene.

5. A process according to any one of claims 1 to 4, wherein R₂ is C₁-C₂alkyl or is phenyl that is unsubstituted or substituted by methyl, methoxy or by chlorine, and is preferably ethyl or phenyl.

6. A process according to any one of claims 1 to 5, wherein the silylating agent is an alkylsilyl halide, alkylsilylamine, hexaalkyl-disilazane or N-silylacetamide.

7. A process according to any one of claims 1 to 6, wherein the silylating agent corresponds to formula 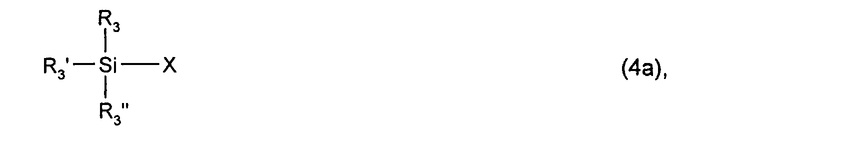 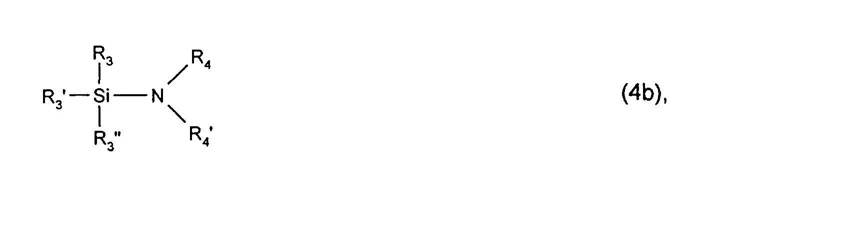 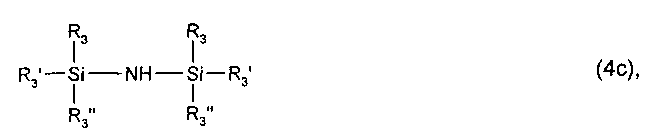 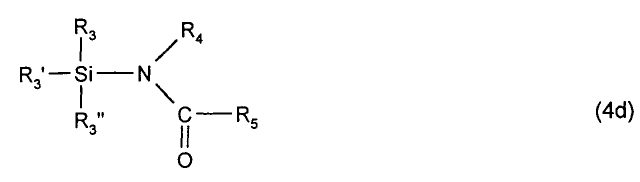 or 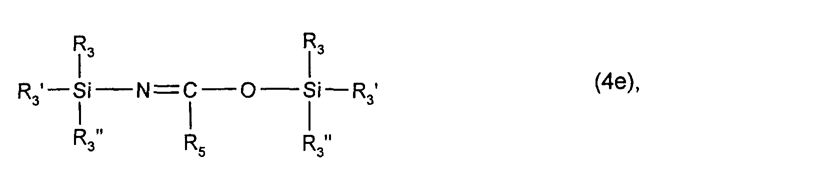 wherein X is halogen, R₃, R₃' and R₃" are each independently of the others C₁-C₄alkyl, R₄ and R₄' are each independently of the other hydrogen or C₁-C₄alkyl and R₅ is C₁-C₄alkyl.

8. A process according to any one of claims 1 to 7, wherein the silylating agent is trimethylsilyl chloride, hexamethyldisilazane, N-trimethylsilyl-N,N-diethylamine, N,O-bis(trimethylsilyl)acetamide or N-trimethylsilyl-N-methylacetamide or a mixture of trimethylsilyl chloride and hexamethyldisilazane.

9. A process according to any one of claims 1 to 8, wherein the silylation of the ethylenically unsaturated urethane is performed in an aprotic organic solvent at a temperature of from 15 to 50°C under a protective gas atmosphere.

10. A process according to any one of claims 1 to 9, wherein the ethylenically unsaturated N-silyl-urethane is converted thermally into the ethylenically unsaturated isocyanate directly, without working up.

11. A process according to any one of claims 1 to 10, wherein the thermolysis of the ethylenically unsaturated N-silyl-urethane to form the ethylenically unsaturated isocyanate is performed in an aprotic solvent under a protective gas atmosphere.

12. A process according to claim 1 for the preparation of compounds of formula (1) wherein x is the number 1, B is a radical of formula (2) wherein B₁ is linear or branched C₂-C₈alkylene and R₁ is hydrogen or methyl, which process comprises reacting a compound of formula (3) wherein R₂ is ethyl or phenyl and R₁, B and x are as defined above, with a silylating agent selected from the group alkylsilyl halides, alkylsilylamines, hexaalkyl-disilazanes and N-silylacetamides to form a compound of formula (5) and directly, without working up, subjecting that compound to thermolysis at a temperature of from 70 to 120°C.

13. A process according to claim 12, wherein R₂ is phenyl and the thermolysis is carried out at a temperature of from 80 to 100°C.

14. A process for the preparation of compounds of formula 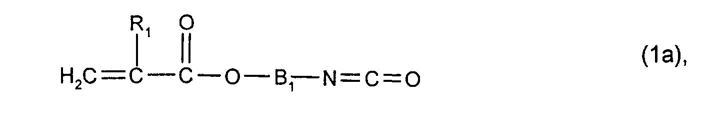 wherein R₁ is hydrogen or methyl and B₁ is linear or branched C₂-C₁₂alkylene that is uninterrupted or is interrupted by one or more oxygen atoms, which process comprises
(a) reacting a compound of formula 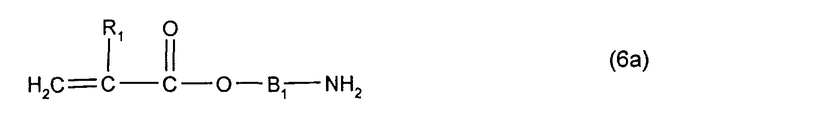 or an acid addition salt thereof, wherein R₁ and B₁ are each as defined above, with a haloformic acid ester of formula 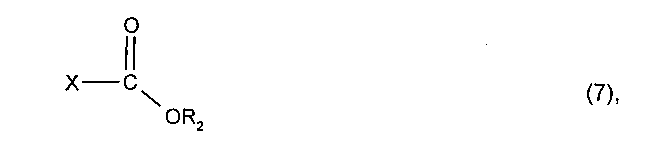 wherein X is halogen and R₂ is C₁-C₄alkyl or is phenyl that is unsubstituted or substituted by C₁-C₄alkyl, C₁-C₄alkoxy or by halogen, to form a compound of formula 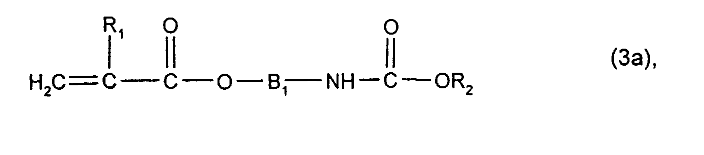
(b) converting the urethane compound obtainable according to (a), with the aid of a silylating agent, into a compound of formula 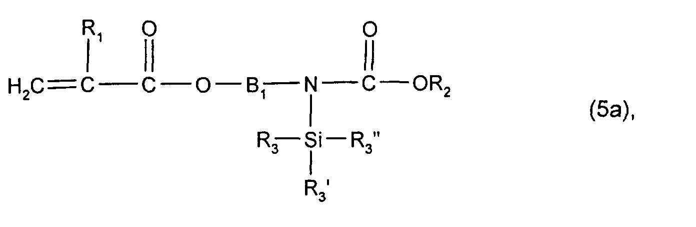 wherein R₃, R₃' and R₃" are each independently of the others C₁-C₄alkyl and the variables R₁, R₂ and B₁ are as defined above, and
(c) converting the N-silylurethane compound obtainable according to (b), by means of thermolysis at a temperature of from 70 to 120°C, into a compound of formula (1a).

15. A process for the preparation of compounds of formula 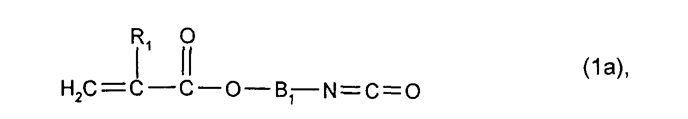 wherein R₁ is hydrogen or methyl and B₁ is linear or branched C₂-C₁₂alkylene that is uninterrupted or is interrupted by one or more oxygen atoms, which process comprises
(a) reacting a compound of formula 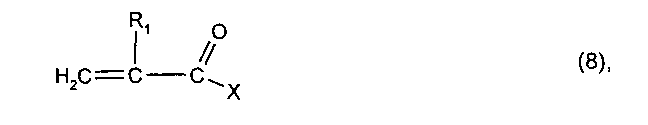 wherein R₁ is as defined above and X is halogen, with a compound of formula 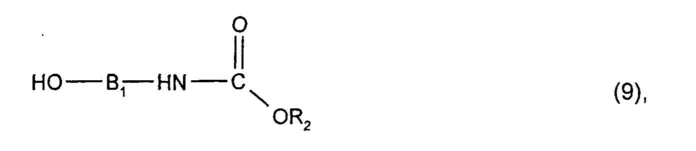 wherein R₂ is C₁-C₄alkyl or is phenyl that is unsubstituted or substituted by C₁-C₄alkyl, C₁-C₄alkoxy or by halogen and B₁ is as defined above, to form a compound of formula 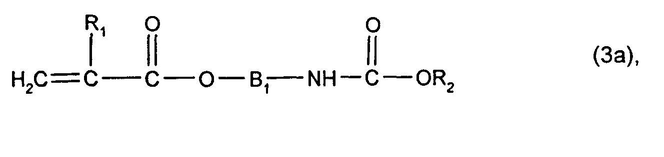
(b) converting the urethane compound obtainable according to (a), with the aid of a silylating agent, into a compound of formula 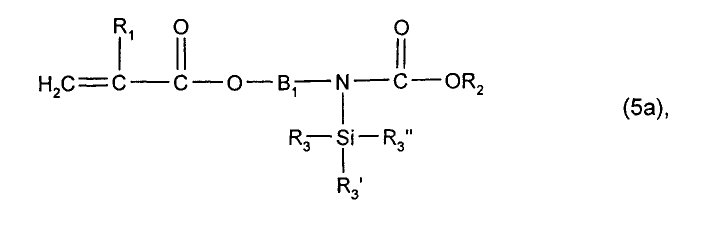 wherein R₃, R₃' and R₃" are each independently of the others C₁-C₄alkyl and the variables R₁, R₂ and B₁ are as defined above, and
(c) converting the N-silylurethane compound obtainable according to (b), by means of thermolysis at a temperature of from 70 to 120°C, into a compound of formula (1a).

## Revendications

1. Un procédé de préparation d'isocyanates éthyléniquement insaturés de formule 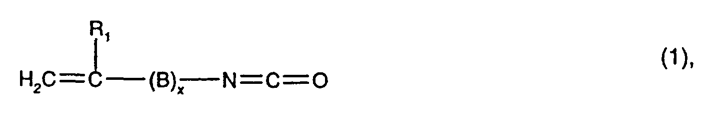 où
R₁ signifie l'hydrogène ou un groupe méthyle,
x signifie le nombre 0 ou 1,
B signifie un groupe phénylène ou C₇-C₁₂-aralkylène éventuellement substitués par un groupe C₁-C₄-alkyle ou C₁-C₄-alcoxy ou bien signifie un radical de formule 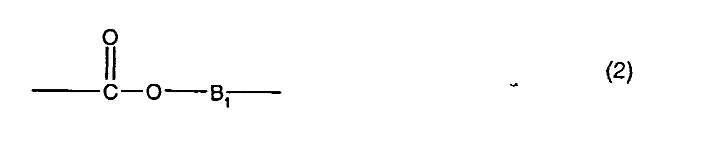 et B₁ signifie un groupe C₂-C₁₂-alkylène linéaire ou ramifié éventuellement interrompu par un ou plusieurs atomes d'oxygène, **caractérisé en ce qu'**on transforme un uréthane éthyléniquement insaturé de formule 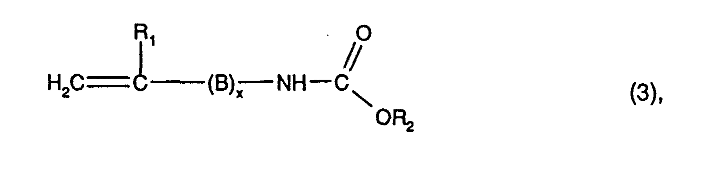 où R₂ signifie un groupe C₁-C₄-alkyle ou signifie un groupe phényle éventuellement substitué par un groupe C₁-C₄-alkyle, C₁-C₄-alcoxy ou par un halogène et les variables R₁, B et x ont la signification donnée plus haut, avec un agent de silylation, en N-silyl-uréthane éthyléniquement insaturé de formule 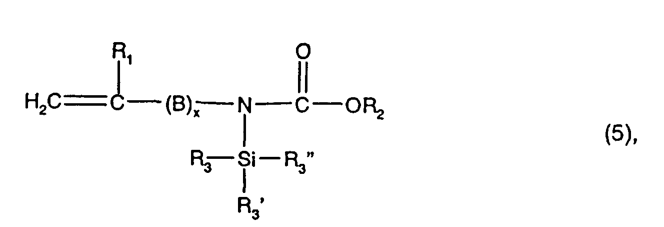 où R₃, R₃' et R₃" signifient chacun indépendamment des autres, un groupe C₁-C₄-alkyle et les variables R₁, R₂, B et x ont la signification donnée plus haut, et on transforme ce composé à une température comprise entre 70° et 120°C en isocyanate éthyléniquement insaturé de formule (1).

2. Un procédé selon la revendication 1, **caractérisé en ce que** x signifie le nombre 1.

3. Un procédé selon la revendication 1 ou 2, **caractérisé en ce que** x signifie le nombre 1 et B signifie un radical de formule (2) indiqué à la revendication 1.

4. Un procédé selon l'une quelconque des revendications 1 à 3, **caractérisé en ce que** B₁ signifie un groupe C₂-C₈-alkylène linéaire ou ramifié, de préférence un groupe C₂-C₄-alkylène linéaire.

5. Un procédé selon l'une quelconque des revendications 1 à 4, **caractérisé en ce que** R₂ signifie un groupe C₁-C₂-alkyle ou signifie un groupe phényle qui est non substitué ou substitué par un groupe méthyle, méthoxy ou par le chlore, et signifie de préférence un groupe éthyle ou phényle.

6. Un procédé selon l'une quelconque des revendications 1 à 5, **caractérisé en ce que** l'agent de silylation est un halogénure d'alkylsilyle, une alkylsilylamine, un hexaalkyl-disilazane ou un N-silylacétamide.

7. Un procédé selon l'une quelconque des revendications 1 à 6, **caractérisé en ce que** l'agent de silylation correspond à la formule 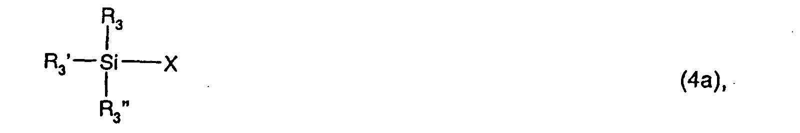 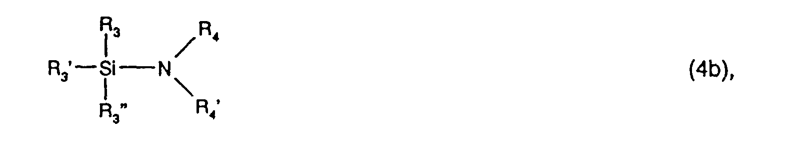 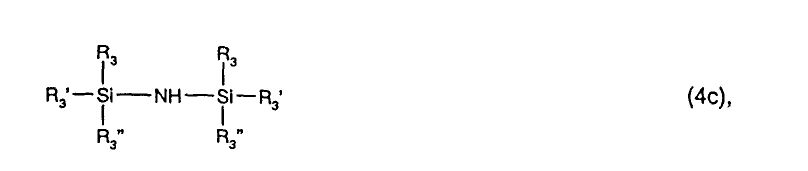 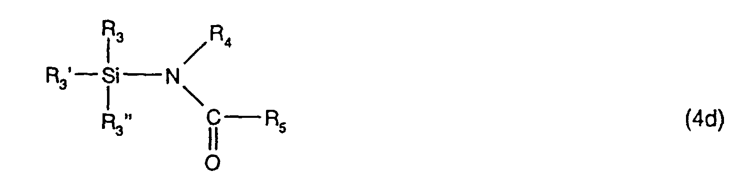 ou 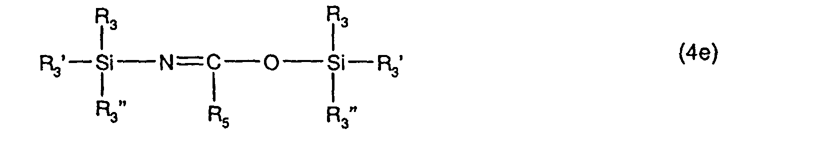 où X signifie un halogène, R₃, R₃' et R₃" signifient chacun indépendamment des autres, un groupe C₁-C₄-alkyle, R₄ et R₄' signifient chacun indépendamment de l'autre, l'hydrogène ou un groupe C₁-C₄-alkyle et R₅ signifie un groupe C₁-C₄-alkyle.

8. Un procédé selon l'une quelconque des revendications 1 à 7, **caractérisé en ce que** l'agent de silylation est le chlorure de triméthylsilyle, l'hexaméthyldisilazane, la N-triméthylsilyl-N,N-diéthylamine, le N,O-bis(triméthylsilyl)acétamide ou le N-triméthylsilyl-N-méthylacétamide ou un mélange de chlorure de triméthylsilyle et d'hexaméthyldisilazane.

9. Un procédé selon l'une quelconque des revendications 1 à 8, **caractérisé en ce que** la silylation de l'uréthane éthyléniquement insaturé est effectuée dans un solvant organique aprotique à une température comprise entre 15 et 50°C sous atmosphère de gaz protecteur.

10. Un procédé selon l'une quelconque des revendications 1 à 9, **caractérisé en ce que** le N-silyl-uréthane éthyléniquement insaturé est transformé thermiquement en isocyanate éthyléniquement insaturé directement, sans traitement ultérieur.

11. Un procédé selon l'une quelconque des revendications 1 à 10, **caractérisé en ce que** la thermolyse du N-silyl-uréthane éthyléniquement insaturé pour former l'isocyanate éthyléniquement insaturé est effectuée dans un solvant aprotique sous atmosphère de gaz protecteur.

12. Un procédé selon la revendication 1 pour la préparation des composés de formule (1), où x signifie le nombre 1, B signifie un radical de formule (2) où B₁ signifie un groupe C₂-C₈-alkylène linéaire ou ramifié et R₁ signifie l'hydrogène ou un groupe méthyle, **caractérisé en ce qu'**on fait réagir un composé de formule (3) où R₂ signifie un groupe éthyle ou phényle et R₁, B et x ont la signification donnée plus haut, avec un agent de silylation choisi parmi le groupe comprenant les halogénures d'alkylsilyle, les alkylsilylamines, les hexaalkyl-disilazanes et les N-silylacétamides pour former un composé de formule (5) et on soumet ce composé à une thermolyse à une température comprise entre 70 et 120°C, directement, sans traitement ultérieur.

13. Un procédé selon la revendication 12, **caractérisé en ce que** R₂ signifie un groupe phényle et la thermolyse a lieu à une température comprise entre 80 et 100°C.

14. Un procédé de préparation des composés de formule 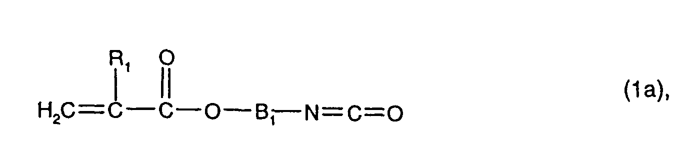 où R₁ signifie l'hydrogène ou un groupe méthyle et B₁ signifie un groupe C₂-C₁₂-alkylène linéaire ou ramifié éventuellement interrompu par un ou plusieurs atomes d'oxygène, **caractérisé en ce que**
(a) on fait réagir un composé de formule 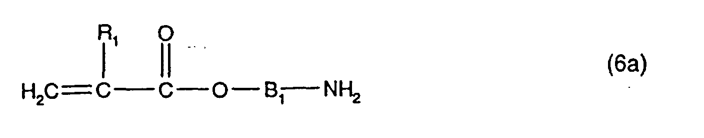 ou un de ses sels d'addition d'acides, où R₁ et B₁ ont chacun la signification donnée plus haut, avec un ester de l'acide halogéno-formique de formule 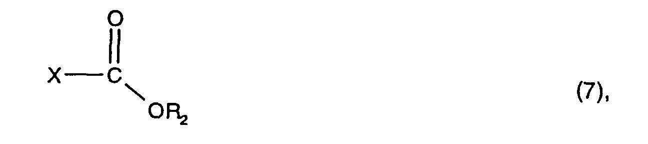 où X signifie un halogène et R₂ signifie un groupe C₁-C₄-alkyle ou signifie un groupe phényle éventuellement substitué par un groupe C₁-C₄-alkyle, C₁-C₄-alcoxy ou par un halogène, pour former un composé de formule 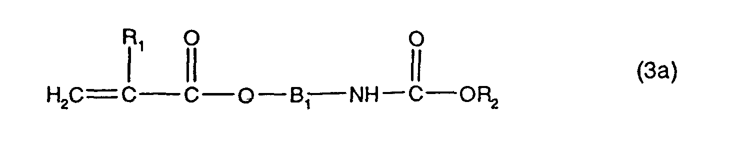
(b) on transforme le composé uréthane pouvant être obtenu selon (a), avec un agent de silylation, en composé de formule 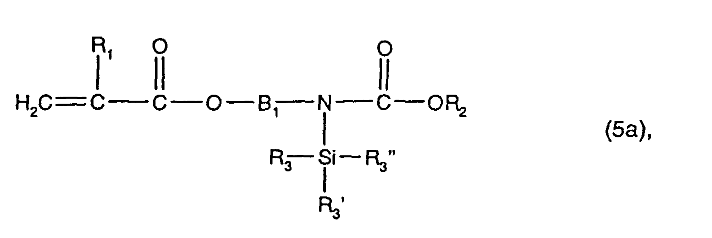 où R₃, R₃' et R₃" signifient chacun indépendamment des autres, un groupe C₁-C₄-alkyle et les variables R₁, R₂ et B₁ ont la signification donnée plus haut, et
(c) on transforme le composé N-silyluréthane pouvant être obtenu selon (b), par thermolyse à une température comprise entre 70 et 120°C, en composé de formule (1a).

15. Un procédé de préparation des composés de formule 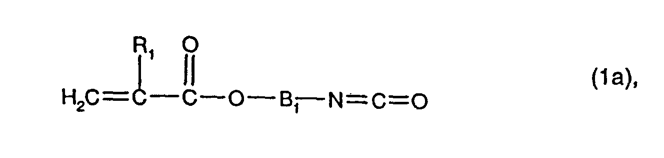 où R₁ signifie l'hydrogène ou un groupe méthyle et B₁ signifie un groupe C₂-C₁₂-alkylène linéaire ou ramifié qui est éventuellement interrompu par un ou plusieurs atomes d'oxygène, **caractérisé en ce que**
(a) on fait réagir un composé de formule 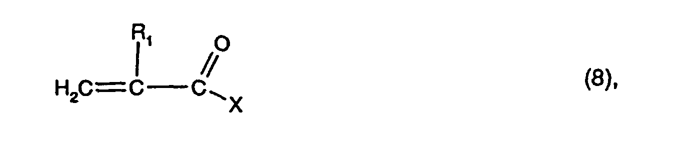 où R₁ a la signification donnée plus haut et X signifie un halogène, avec un composé de formule 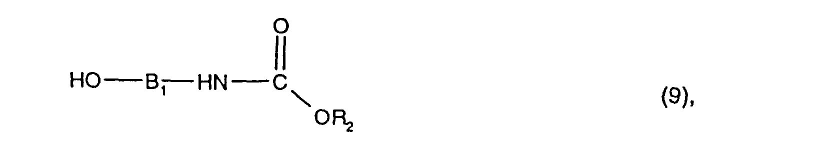 où R₂ signifie un groupe C₁-C₄-alkyle ou signifie un groupe phényle éventuellement substitué par un groupe C₁-C₄-alkyle, C₁-C₄-alcoxy ou par un halogène et B₁ a la signification donnée plus haut, pour former un composé de formule 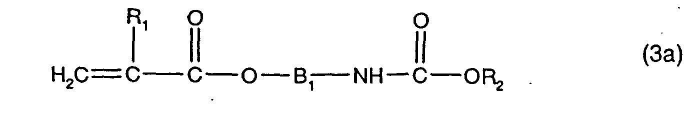
(b) on transforme le composé uréthane pouvant être obtenu selon (a), avec un agent de silylation, en composé de formule 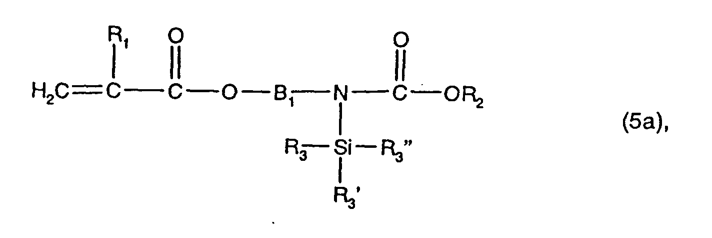 où R₃, R₃' et R₃" signifient chacun indépendamment des autres, un groupe C₁-C₄-alkyle et les variables R₁, R₂ et B₁ ont la signification donnée plus haut, et
(c) on transforme le composé N-silyluréthane pouvant être obtenu selon (b), par thermolyse à une température comprise entre 70 et 120°C, en composé de formule (1a).
